# EUROPEAN PATENT SPECIFICATION

(11) **EP 3 993 842 B1**
(45) Date of publication and mention of the grant of the patent: **04.12.2024**
(21) Application number: 20835000.9
(22) Date of filing: 05.06.2020
(51) Int. Cl.: A61L 2/10, A61L 2/24, A61L 2/26, A42B 3/00

(54) **HELMET BAG WITH UV SANITIZATION FUNCTION**
HELMBEUTEL MIT UV-DESINFEKTIONSFUNKTION
SAC POUR CASQUE AVEC FONCTION DE DÉSINFECTION UV

(30) Priority: 01.07.2019 MY PI2019003815
(43) Date of publication of application: 11.05.2022
(73) Proprietor: Lee, Yeat Hun, Pulau Pinang, 10050 (MY)
(72) Inventor: Lee, Yeat Hun, Pulau Pinang, 10050 (MY)
(74) Representative: Cabinet Chaillot
(86) International application number: PCT/MY2020/000006
(87) International publication number: WO 2021/002741

(56) References cited:
- CN-Y- 200 973 845
- CN-Y- 201 147 513
- KR-B1- 101 530 277
- KR-B1- 101 770 090
- KR-B1- 101 898 429
- KR-B1- 101 920 377
- US-A1- 2014 365 360
- US-A1- 2018 154 028
- US-A1- 2018 154 028
- US-A1- 2019 162 471

## Description

### FIELD OF THE INVENTION

The present invention relates to the field of sanitization and disinfection of a helmet and in particular it relates to UV sanitizing and disinfecting of a motorcycle helmet.

### BACKGROUND OF THE INVENTION

Helmets are worn mostly by motorcyclists, cyclists and workmen.

With constant use, sweat accumulates in the helmet and causes bacteria, genomes, pathogens and other like microorganisms to grow and proliferate inside the helmet. This creates an unhealthy environment inside the helmet and is a health hazard to the wearer unless it is constantly cleaned, disinfected and sanitized.

There are many known devices which are used in cleaning, deodorizing, sanitizing, disinfecting and even sterilizating of motorcycle helmets.

Because of sweat, primarily, the process of cleaning involves drying the inner linings of the helmet, accompanied by the process of containing and removing the bacteria, microbes and pathogens residing inside the helmet or at the least rendering them harmless.

To achieve this, blowing and managing the air flow inside the helmet to dry it, is necessary, accompanied by applying means to disinfect and sanitize the inside of the helmet.

Simple methods current adopted are to manually blow air into the helmet to dry the inner linings and also manually apply or spray disinfectants and deodorizers to sanitize the inside of the helmet.

For some, the cleaning processes may be built in the helmet itself, whereas for others, more elaborate cleaning processes are built into a separate receptacle or housing to perform the tasks.

Prior art devices having inbuilt cleaning and sanitizing functions inside the helmet itself is difficult non practical, inefficient and very limited. Except for improving ventilation and air circulation within the helmet with perhaps adding extra linings with anti-septic pads or using antiseptic swipes or sprays, it is difficult to provide for effective sanitization and disinfection and at the same time provide protection and safety for the wearer, because the more effective methods of sanitization, sterilization and disinfection like applying UV, ozone, liquid and chemical cleansing methods can be hazardous and are not practical or possible to be built into the helmet itself, but have to require a separate housing, container, machine or receptacle to perform the cleaning task.

This means that it is not so convenient and the wearer has to leave his helmet in a separate machine, housing or receptacle quite often, placed away from his vehicle, purposely for the cleaning process.

Examples of such prior art devices are found in Patent No. CN 107495520 which discloses a Solar Power Generation Helmet with Disinfection Function, which belongs to the first type that has built in disinfection functions in the helmet itself, which is although convenient, but impractical or inefficient and can be harmful and hazardous for the wearer, and whereas in Patent No. KR101770090 which describes an Apparatus and Method for Sterilizing and Disinfecting of Helmet and CN 200973845 which discloses a UV Radiation Ozone Machine for Sterilizing and Cleaning Crash Helmet, both belonging to the second type that require expensive, bulky and elaborate machine setups. US 2018/154028 discloses a soft bag with a built-in sanitizing function including a UV-C lamp, an electrical fan and electric control means.

However, such elaborate measures used in existing prior art devices for the process of cleaning, sanitizing, disinfecting and sterilizing the helmet especially by using ozone, liquids and chemicals are quite unnecessary, inconvenient and not cost effective.

Besides, in the case of a motorcycle helmet, since there is no actual requirement to disinfect and sterilize, to the extent of total annihilation of all microorganisms, but rather it is adequate to just sanitize to disinfect the bacteria, genomes, pathogens and such microbes to render them harmless, and most importantly to keep the helmet constantly dry from the accumulated sweat.

There is a need to have a system and apparatus for cleaning, deodorizing, sanitizing and disinfecting a motor cycle helmet in a more convenient, safe, efficient and more economical manner with the use of a separate case or receptacle that is handy, easily portable, and also provides convenience and safety for the wearer.

### SUMMARY OF THE INVENTION

It is an objective of the present invention to provide for a safe and efficient means for cleaning a motor cycle helmet.

It is another object of the present invention to provide for an economical and convenient means of sanitizing a motorcycle helmet.

It is a further object of the present invention to provide for a handy and portable means of sanitizing and disinfecting a motor cycle helmet.

It is yet a further object of the present invention to provide a means of using UV sanitization on to helmets.

UV sanitizing is more suitable for cleaning and disinfecting helmets, because UV rays contain a very high amount of energy that can penetrate cells, reaching the DNA where they can cut the genome and undermine its functionality. With just a few minutes of exposure, an auto-destruction program (known as apoptosis) is normally initiated in the cell.

However even at lower exposures, where the cell may survive and simply just be damaged, they can be rendered harmless.

Infact, this disinfection method uses short-wavelength ultraviolet (UV-C) light to kill or inactivate microorganisms by destroying nucleic acids and disrupting their DNA, leaving them unable to perform their vital cellular functions.

The present invention is a bag made specifically to fit a motorcycle helmet comprising EVA material fixed with a built in UV-C lamp to assist in sanitizing the helmet and includes a fan to effectively direct appropriate air flow within the helmet and coupled with a replace-ably consumable scent pouch for assisting in deodorizing and disinfecting the said helmet controlled by an electrical control means activated by sensors within an electrical circuit to provide for automatic cut off and safe use.

The bag may also be a motorcycle pannier or a saddle bag that is conveniently slung over the vehicle and is usually carried along to be ready for use anytime as storage for the helmet as well as for cleaning and sanitizing the helmet, when the rider is not wearing it.

It is preferably made of hard shell plastic EVA material that takes the shape of the motorcycle helmet with cleaning, sanitizing and disinfecting functions built in the bag itself.

### BRIEF DESCRIPTION OF THE DRAWINGS

A preferred embodiment of the present invention is hereinafter described by way of example only with reference to the drawings wherein:-
Figure 1 is a perspective view of the helmet bag in accordance with the present invention.
Figure 2 is a perspective view of the helmet bag showing the orientation and configuration of the helmet in place within the said bag during the cleaning process in accordance with the present invention.
Figure 3 is an exploded view of the helmet bag illustrating the assembly of the various parts within the said bag in accordance to the present invention.
Figure 4 shows the air-circulation within the helmet bag in accordance to the present invention with Figure 4(a) showing an Xray view of the helmet bag illustrating the air flow within the helmet bag itself and Figure (b) showing the bottom view of the helmet bag, for where the air will exit through the air vents located at the bottom of the helmet bag.
Figure 5(a) is an Xray view showing a helmet retained by the foam retainer inside the helmet bag.
Figure 5(b) is another Xray view of the helmet bag illustrating how different size helmets may fit inside the said bag.

### DETAILED DESCRIPTION OF THE PREFERRED EMBODIMENT

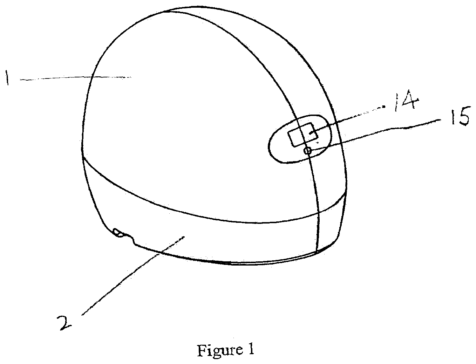

Referring to Figure 1 the helmet bag of the present invention is shown with a top cover (1) in a closed position with a bottom case (2) of the said bag.

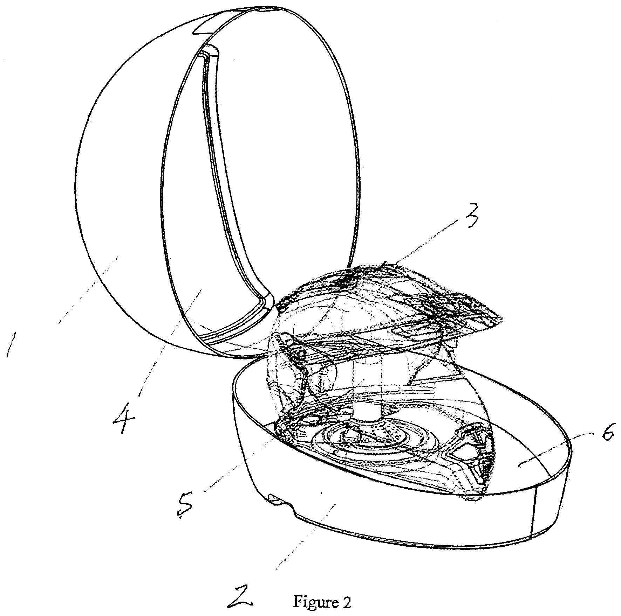

Figure 2 illustrates the orientation and configuration of a helmet (3) which is held in place on top by a foam retainer (4) wherein the helmet (3) covers an UV-C lamp (5) and rests on a pallet (6) which in turn sits on the bottom case (2) within the helmet bag with the top cover (1) open in preparation for the cleaning, disinfection and sanitization process in accordance with the present invention.

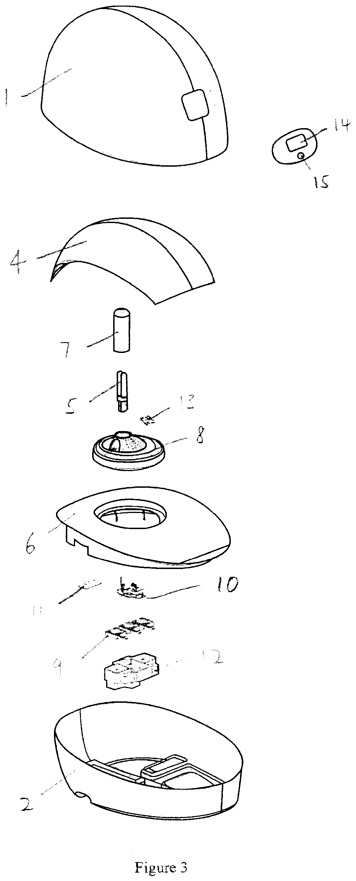

Figure 3 is an exploded view of the helmet bag which comprises;
(i) top cover (1) made preferably of hard shell plastic EVA material that takes the shape of the helmet which is releasably hinged to;
(ii) bottom case (2) of similar material and built in within;
(iii) UV-C lamp (5) for UV disinfection and sanitization, mounted on;
(iv) pallet (6) with lamp cover (7) on top of it and held by a lamp holder (8) with a plurality of ventilation holes for attaching the lamp (5) to the said pallet (6) and for air to flow through them. The pallet (6) sits on the bottom case (2) and includes
(v) a plurality of electrical fans (9) for circulating the air within, attached on the bottom of the said pallet (6) together with;
(vi) an electrical control means (10) activated by a set of sensors (11) forming a electronic circuit mounted on a circuit holder (12) connected to a power supply to control the automatic cutoff and on/off switching of the cleaning process, along with;
(vii) a scent pouch (13) to assist in deodorizing and disinfecting the helmet (3) within the said bag.

The scent pouch (13) is a consumable that may be replaced when needed.

The electrical control means (10) is connected to an external LCD indicator (14) with a manual switch (15) which may be located anywhere on the outside of the bag top cover (1) as shown in Figure 1 and Figure 3 to monitor the current timing and status plus manual on/off switching of the cleaning process.

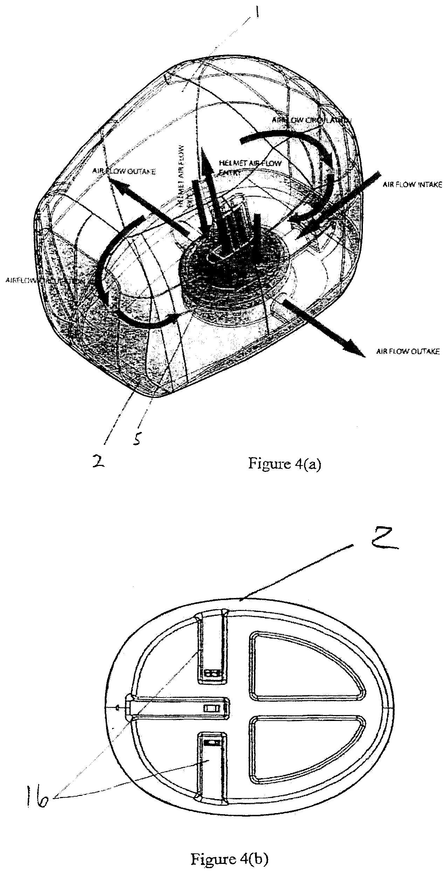

Referring to Figure 3 and Figure 4 when operation begins, air is drawn in from the atmosphere with the help of an inlet fan (9) at the centre and air is drawn out by a pair of exhaust fans (9) at each end with appropriate air flow circulating within the helmet bag as shown in Figure 4 (a) (where the direction of the airflow is illustrated) in order to dry the insides of the helmet (3) during the cleaning process.

The process of cleaning, disinfecting, sanitizing, drying and deodorizing begins with first placing the helmet (3) inside the bag sitting on the pallet (6) which is retained firmly by the retainer (4) and then upon closing the top cover (1) to seal the helmet (3) within. The helmet (3) should be covered, with the UV-C lamp (5) in the middle, in order to effectively sanitize the interior of the helmet (3), and would automatically be switched on for the cleaning process, when the helmet (3) is firmly seated in place.

The cleaning process is controlled by the electrical control means (10) activated by sensors (11) to ensure that the top cover (1) is properly closed and the bag is properly sealed before turning on the UV-C lamp (5) and the fans (9), so as to ensure safety, because long term exposure of UV light can be harmful and hazardous.

To begin with, the sensors (11) will start operating with one of the light sensors (11) detecting any ambient light source. For if the bag does not close under normal environment, the ambient light sensor (11) will pickup some light. The other sensor (11) is to detect whether if the bag is properly closed and the environment is safe, before activating the switch to start the cleaning process.

After completing this safety check, both the UV lamp (5) and the fans (11) will then proceed to operate.

The UV light will only turn on for about 15-20 minutes to complete the sanitizing process, but the fans (11) will continue blowing to circulate the air until the insides of the helmet (3) and the inner linings are completely dry. Once the whole cycle is completed, the circuit will automatically shut off.

If during the cleaning process, the bag is inadvertently opened, the circuit will automatically shut down the UV light immediately, so as to prevent unnecessary exposure and risks.

The air is then drawn in by the inlet fan (9) via the air flow intake as illustrated in Figure 4(a) and continues to circulate the air within to dry the insides of the helmet (3) to exit via the air flow outtake and through the exhaust air vents (16) located at the bottom case (2) as shown in Figure 4(b).

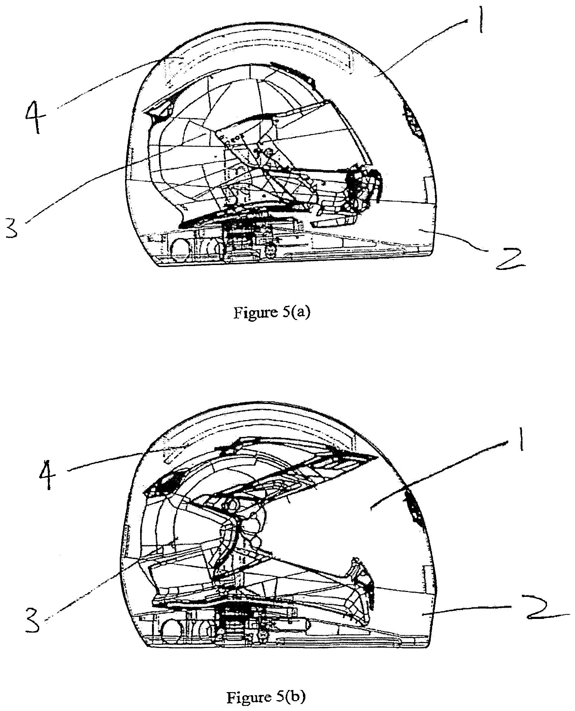

Different sizes of helmets (3) may be fitted inside the helmet bag and retained by a customized foam retainer (4) which fits as shown in Figure 5(a) and Figure 5(b). It will be obvious to those having skill in the art that many changes and variations may be made to the details of the above described embodiment without departing from the scope of the invention. The scope of the present invention should therefore be determined only by the following claims.

## Claims

1. A helmet bag which has a top cover (1) is releasably hinged to a bottom case (2) **characterized in that**, it has built-in sanitizing functions for a helmet (3) within the bag, comprising :-
(i) an UV-C lamp (5) for UV disinfection and sanitization, mounted on;
(ii) a pallet (6) with a lamp cover (7) on top of it and held by a lamp holder (8) which has a plurality of ventilation holes, for attaching the lamp (5) to the said pallet (6) and for air to flow through them, wherein the pallet (6) sits on the bottom case (2) that includes;
(iii) a plurality of electrical fans (9) for circulating the air within,
attached onto the bottom of the said pallet (6) together with;
(iv) an electrical control means (10) activated by a set of sensors (11) forming an electronic circuit connected to a power supply to control the automatic cutoff and on/off switching of the cleansing process, coupled with;
(v) a scent pouch (13) to assist in deodorizing and disinfecting the helmet (3) within the said bag.

2. The helmet bag as claimed in Claim 1 further **characterized in that** the electronic control means (10), sensors (11) and electronic circuit are connected to an external LCD indicator (14) with a manual switch (15) which may be located anywhere on the outside of the bag top cover (1) to monitor current timing and status plus manual on/off switching of the cleaning process.

3. The helmet bag as claimed in Claim 1 wherein the helmet (3) can be of difference sizes by using customized foam retainers (4).

4. The helmet bag as claimed in Claim 1 wherein the scent pouch (13) is a consumable that may be replaced when needed.

5. The helmet bag as claimed in Claim 1 wherein the helmet (3) should cover, with the UV-C lamp (5) in the middle, in order to effectively sanitize the interior of the helmet (3), and would automatically be switched on for the cleaning process, when the helmet (3) is firmly seated in place.

6. The helmet bag as claimed in Claim 1 wherein the UV lamp (5) and the fans (9) will proceed to operate only after a safety check is done by the sensors (11) of the electrical control means (10), and electronic circuits which will monitor the cleaning process, to provide for automatic cut off to prevent unnecessary UV exposure and to ensure safety.

7. The helmet bag as claimed in Claim 1 wherein the plurality of electrical fans (9) comprises (i) an inlet fan (9), that draws in the air from the atmosphere (ii) an exhaust fan (9) that draws out the air through outlet air vents (16), located at the bottom case (2) after appropriate air flow has circulated within the helmet bag, in order to dry the insides of the helmet (3) during the cleansing process.

8. A method of sanitizing a helmet (3) by using a helmet bag which has a top cover (1) is releasably hinged to a bottom case (2) **characterized in that**, built in within the bag, it comprises:-
(i) an UV-C lamp (5) for UV disinfection and sanitization, mounted on;
(ii) a pallet (6) with a lamp cover (7) on top of it and held by a lamp holder (8) which has a plurality of ventilation holes, for attaching the lamp (5) to the said pallet (6) and for air to flow through them, wherein the pallet (6) sits on the bottom case (2) that includes;
(iii) a plurality of electrical fans (9) for circulating the air within, attached onto the bottom of the said pallet (6) together with;
(iv) an electrical control means (10) activated by a set of sensors (11) forming an electronic circuit connected to a power supply to control the automatic cutoff and on/off switching of the cleansing process, coupled with;
(v) a scent pouch (13) to assist in deodorizing and disinfecting the helmet (3) within the said bag.

## Patentansprüche

1. - Helmtasche, die eine obere Abdeckung (1) aufweist, die lösbar an einem unteren Gehäuse (2) angelenkt ist, **dadurch gekennzeichnet, dass** sie eingebaute Hygienisierungsfunktionen für einen Helm (3) innerhalb der Tasche aufweist, umfassend:
(i) eine UV-C-Lampe (5) zur UV-Desinfektion und - Hygienisierung, montiert auf;
(ii) einer Palette (6) mit einer Lampenabdeckung (7) an einer Oberseite davon und die von einer Lampenhalterung (8) gehalten wird, die eine Vielzahl von Belüftungslöchern aufweist, zum Anbringen der Lampe (5) an der Palette (6) und damit Luft durch sie hindurchströmen kann, wobei die Palette (6) auf dem unteren Gehäuse (2) sitzt, das Folgendes beinhaltet;
(iii) eine Vielzahl elektrischer Lüfter (9) zum Umwälzen der Luft im Inneren, die an dem Boden der Palette (6) angebracht sind, zusammen mit;
(iv) einer elektrischen Steuereinrichtung (10), die durch einen Satz von Sensoren (11) aktiviert wird, die eine elektronische Schaltung bilden, die mit einer Stromversorgung verbunden ist, um die automatische Abschaltung und das Ein- und Ausschalten des Reinigungsprozesses zu steuern, gekoppelt mit;
(v) einen Duftbeutel (13) zum Unterstützen des Desodorierens und Desinfizierens des Helms (3) in der Tasche.

2. - Helmtasche nach Anspruch 1, **dadurch gekennzeichnet, dass** die elektronische Steuerung (10), die Sensoren (11) und die elektronische Schaltung mit einer externen LCD-Anzeige (14) mit einem manuellen Schalter (15) verbunden sind, der sich an einer beliebigen Stelle an der Außenseite der oberen Abdeckung der Tasche (1) befinden kann, um den aktuellen Zeitpunkt und Status sowie das manuelle Ein- und Ausschalten des Reinigungsprozesses zu überwachen.

3. - Helmtasche nach Anspruch 1, wobei der Helm (3) durch Verwendung von maßgeschneiderten Schaumstoffhaltern (4) unterschiedlich groß sein kann.

4. - Helmtasche nach Anspruch 1, wobei der Duftbeutel (13) ein Verbrauchsmaterial ist, das bei Bedarf ersetzt werden kann.

5. - Helmtasche nach Anspruch 1, wobei der Helm (3) mit der UV-Klammer (5) in der Mitte abgedeckt werden sollte, um das Innere des Helms (3) wirksam zu hygienisieren, und automatisch für den Reinigungsvorgang eingeschaltet werden sollte, wenn der Helm (3) fest an seinem Platz sitzt.

6. - Helmtasche nach Anspruch 1, wobei die UV-Lampe (5) und die Lüfter (9) erst nach einer Sicherheitsprüfung durch die Sensoren (11) der elektrischen Steuervorrichtung (10) und elektronische Schaltungen, die den Reinigungsprozess überwachen, in Betrieb genommen werden, um eine automatische Abschaltung bereitzustellen, um eine unnötige UV-Belastung zu verhindern und Sicherheit zu gewährleisten.

7. - Helmbeutel nach Anspruch 1, die Vielzahl von elektrischen Lüftern (9) umfassend (i) einen Einlasslüfter (9), der die Luft aus der Atmosphäre ansaugt, (ii) einen Auslasslüfter (9), der die Luft durch Auslassluftöffnungen (16) absaugt, die sich an dem unteren Gehäuse (2) befinden, nachdem ein geeigneter Luftstrom in dem Helmbeutel umgewälzt wurde, um die Innenseite des Helms (3) während des Reinigungsprozesses zu trocknen.

8. - Verfahren zum Hygienisieren eines Helms (3) unter Verwendung einer Helmtasche, die eine obere Abdeckung (1) aufweist, die lösbar an einem unteren Gehäuse (2) angelenkt ist, **dadurch gekennzeichnet, dass** sie eingebaut in die Tasche Folgendes umfasst:
(i) eine UV-C-Lampe (5) zur UV-Desinfektion und - Hygienisierung, montiert auf;
(ii) eine Palette (6) mit einer Lampenabdeckung (7) an einer Oberseite davon und die von einer Lampenhalterung (8) gehalten wird, die eine Vielzahl von Belüftungslöchern aufweist, zum Anbringen der Lampe (5) an der Palette (6) und damit Luft durch sie hindurchströmen kann, wobei die Palette (6) auf dem unteren Gehäuse (2) sitzt, das Folgendes beinhaltet;
(iii) eine Vielzahl elektrischer Lüfter (9) zum Umwälzen der Luft im Inneren, die an dem Boden der Palette (6) angebracht sind, zusammen mit;
(iv) eine elektrische Steuereinrichtung (10), die durch einen Satz von Sensoren (11) aktiviert wird, die eine elektronische Schaltung bilden, die mit einer Stromversorgung verbunden ist, um die automatische Abschaltung und das automatische Ein- und Ausschalten des Reinigungsprozesses zu steuern, gekoppelt mit;
(v) einem Duftbeutel (13) zum Unterstützen des Desodorierens und Desinfizierens des Helms (3) in der Tasche.

## Revendications

1. - Sac pour casque qui a une housse supérieure (1) articulée de manière amovible à un boîtier inférieur (2), **caractérisé par le fait qu'**il a des fonctions intégrées d'assainissement pour un casque (3) à l'intérieur du sac, comprenant :
(i) une lampe UV-C (5) pour un assainissement et une désinfection par UV, montée sur ;
(ii) une palette (6) avec un élément de recouvrement de lampe (7) sur celle-ci et maintenu par un support de lampe (8) qui a une pluralité de trous d'aération, pour fixer la lampe (5) à ladite palette (6) et pour permettre à de l'air de s'écouler à travers ceux-ci, la palette (6) reposant sur le boîtier inférieur (2) qui comprend ;
(iii) une pluralité de ventilateurs électriques (9) pour faire circuler l'air à l'intérieur, fixés sur la partie inférieure de ladite palette (6) conjointement avec ;
(iv) un moyen de commande électrique (10) activé par un ensemble de capteurs (11) formant un circuit électronique connecté à une alimentation électrique pour commander la coupure automatique et la commutation marche/arrêt automatique du traitement de nettoyage, couplé à ;
(v) un sachet de parfum (13) pour aider à désodoriser et désinfecter le casque (3) à l'intérieur dudit sac.

2. - Sac pour casque selon la revendication 1, **caractérisé en outre par le fait que** le moyen de commande électronique (10), les capteurs (11) et le circuit électronique sont connectés à un indicateur externe à affichage à cristaux liquides (14) avec un interrupteur manuel (15) qui peut être situé n'importe où à l'extérieur de la housse supérieure de sac (1) pour surveiller un chronométrage actuel et un état actuel ainsi qu'une commutation marche/arrêt manuelle du traitement de nettoyage.

3. - Sac pour casque selon la revendication 1, dans lequel le casque (3) peut être de tailles différentes en utilisant des éléments de retenue en mousse personnalisés (4).

4. - Sac pour casque selon la revendication 1, dans lequel le sachet de parfum (13) est un consommable qui peut être remplacé au besoin.

5. - Sac pour casque selon la revendication 1, dans lequel le casque (3) doit être recouvert, avec la lampe UV-C (5) au milieu, afin d'assainir efficacement l'intérieur du casque (3), et doit être automatiquement mis en marche pour le traitement de nettoyage, lorsque le casque (3) est fermement installé en place.

6. - Sac pour casque selon la revendication 1, dans lequel la lampe UV (5) et les ventilateurs (9) ne fonctionneront qu'après un contrôle de sécurité effectué par les capteurs (11) du moyen de commande électrique (10) et les circuits électroniques qui surveilleront le traitement de nettoyage, afin d'assurer une coupure automatique pour éviter une exposition inutile aux UV et pour garantir la sécurité.

7. - Sac pour casque selon la revendication 1, dans lequel la pluralité de ventilateurs électriques (9) comprennent (i) un ventilateur d'entrée (9), qui aspire l'air depuis l'atmosphère, (ii) un ventilateur de sortie (9) qui expulse l'air par des évents de sortie (16), situés dans le boîtier inférieur (2) après qu'un écoulement d'air approprié a circulé à l'intérieur du sac pour casque, afin de sécher l'intérieur du casque (3) pendant le traitement de nettoyage.

8. - Procédé d'assainissement d'un casque (3) à l'aide d'un sac pour casque qui a une housse supérieure (1) articulée de manière amovible à un boîtier inférieur (2), **caractérisé par le fait que**, intégrés dans le sac, il comprend :
(i) une lampe UV-C (5) pour un assainissement et une désinfection par UV, montée sur ;
(ii) une palette (6) avec un élément de recouvrement de lampe (7) sur celle-ci et maintenu par un support de lampe (8) qui a une pluralité de trous d'aération, pour fixer la lampe (5) à ladite palette (6) et pour permettre à de l'air de s'écouler à travers ceux-ci, la palette (6) reposant sur le boîtier inférieur (2) qui comprend ;
(iii) une pluralité de ventilateurs électriques (9) pour faire circuler l'air à l'intérieur, fixés sur la partie inférieure de ladite palette (6) conjointement avec ;
(iv) un moyen de commande électrique (10) activé par un ensemble de capteurs (11) formant un circuit électronique connecté à une alimentation électrique pour commander la coupure automatique et la commutation marche/arrêt automatique du traitement de nettoyage, couplé à ;
(v) un sachet de parfum (13) pour aider à désodoriser et désinfecter le casque (3) à l'intérieur dudit sac.
